Europäisches Patentamt

European Patent Office

Office européen des brevets

(19)

(11) Veröffentlichungsnummer: **0 045 076**
**B 1**

(12) **EUROPÄISCHE PATENTSCHRIFT**

(45) Veröffentlichungstag der Patentschrift:
17.08.83

(21) Anmeldenummer: 81105882.5

(22) Anmeldetag: 25.07.81

(51) Int. Cl.³: **C 07 D 493/04** // A61K31/34,
(C07D493/04, 307/00)

(54) Verfahren zur Herstellung von 1,4:3,6-Dianhydro-D-glucit-5-nitrat (Isosorbid-5-nitrat).

(30) Priorität: 30.07.80 DE 3028873

(43) Veröffentlichungstag der Anmeldung:
03.02.82 Patentblatt 82/5

(45) Bekanntmachung des Hinweises auf die Patenterteilung:
17.08.83 Patentblatt 83/33

(84) Benannte Vertragsstaaten:
AT BE CH DE FR GB IT LI LU NL SE

(56) Entgegenhaltungen:
DE-A-2 751 934
Carbohydrate res., 2 (1966), S. 122—131

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: Lauer, Karl, Dr.rer.nat., Burgweg 22,
D-6905 Schriesheim (DE)
Erfinder: Kiegel, Einhart, Dr.rer.nat., Gluckstrasse 4,
D-6800 Mannheim 1 (DE)

### Verfahren zur Herstellung von 1,4:3,6-Dianhydro-D-glucit-5-nitrat (Isosorbid-5-nitrat)

Die Erfindung betrifft ein neues Verfahren zur großtechnischen und wirtschaftlichen Herstellung von 1,4:3,6-Dianhydro-D-glucit-5-nitrat (internationale Kurzbezeichnung Isosorbid-5-nitrat), das zur Behandlung coronarer Herzkrankheiten, z. B. Angina pectoris, verwendet werden kann.

Aus der Literatur sind eine Reihe von Verfahren bekannt, die sich nach vier Grundprinzipien klassifizieren lassen:

a) Die direkte Nitrierung von Isosorbid wird in Photochem., Photobiol. 4, 657 (1965) und in DE-OS 2 221 080 beschrieben. Sie führt in schlechter Ausbeute zu Gemischen von Isosorbid-2-nitrat und Isosorbid-5-nitrat, die dann durch kostspielige und zeitraubende Verfahren getrennt werden müssen. Darüber hinaus besteht bei dieser Methode die Gefahr, daß das explosive Isosorbid-2,5-dinitrat mitentsteht. (Unter »Nitrierung« wird hier und im folgenden die Bildung eines Salpetersäureesters verstanden.)

b) Aus diesem Grunde ist auch das in Org. Magn. Resonance 3, 693 (1971) und Can. J. Chem. 45, 2192 (1967) beschriebene Verfahren, bei dem zunächst Isosorbid-2,5-dinitrat hergestellt und anschließend partiell hydrolysiert wird, für ein großtechnisches Verfahren nicht geeignet.

c) In DE-OS 2 903 927 ist ein Verfahren zur Herstellung der beiden isomeren Isosorbidmononitrate beschrieben, das von Isomannid ausgeht. Dieses wird beispielsweise zunächst mit Tosylchlorid zum Isomannid-2-tosylat verestert, das dann (z. B.) mit Kaliumbenzoat in einer $S_N2$-Reaktion unter Walden'scher Umkehr zum Isosorbid-2-benzoat umgesetzt wird. Die ungeschützte Hydroxylgruppe wird nunmehr mit $HNO_3$ nitriert, die Benzoyl-Gruppe durch partielle Verseifung abgespalten und Isosorbid-5-nitrat erhalten. Nachteilig erweisen sich bei diesem Verfahren das Auftreten verschiedener, nur noch mühsam regenerierbarer Lösungs- und Extraktionsmittelgemische, das ausbeutemindernde Einschieben einer vierten Stufe und die komplizierten Reinigungsoperationen, insbesondere auf der letzten Stufe. Besonders die auftretenden Lösungsmittelgemische und die aufwendigen Reinigungsschritte stehen einer großtechnischen Anwendung dieses Verfahrens im Wege.

d) Einige der dem Verfahrensprinzip nach Punkt c) anhaftenden Nachteile fallen weg, wenn man statt von Isomannid von Isosorbid ausgeht. Ein solches Verfahren ist in der DE-OS 2 751 934 sowie der entsprechenden US-PS 4 065 488 beschrieben. Isosorbid wird hier zunächst mit einem Niedrigalkansäureanhydrid oder Niedrigalkansäurechlorid oder -bromid, insbesondere Essigsäureanhydrid, zu einem Gemisch aus Isosorbid, Isosorbid-2-acylat, Isosorbid-5-acylat und Isosorbid-2,5-diacylat verestert. In einer zweiten Stufe wird das Isosorbid aus dem Gemisch extrahiert, um in der nachfolgenden Nitrierung die Bildung des explosionsgefährlichen Isosorbid-2,5-dinitrats zu verhindern. In der dritten Stufe wird sodann das Gemisch aus Isosorbid-2-acylat, Isosorbid-5-acylat und Isosorbid-2,5-diacylat mit Salpetersäure nitriert und das erhaltene Gemisch aus Isosorbid-2-acylat-5-nitrat, Isosorbid-5-acylat-2-nitrat und Isosorbid-2,5-diacylat partiell hydrolysiert, so daß ein Gemisch aus Isosorbid-2-nitrat und Isosorbid-5-nitrat und Isosorbid entsteht. Durch Auskristallisieren aus geeigneten Lösungsmitteln wird schließlich das Isosorbid-2-nitrat isoliert. Isosorbid-5-nitrat entsteht bei diesem Prozeß offenbar nur untergeordnet.

Von Buck et al., Carbohydrate Research, 2 (1966), S. 122—131 ist bekannt, Isosorbid in Pyridin, das gleichzeitig als Lösungsmittel, Säureakzeptor und Katalysator wirkt, mit 1 Mol Acetanhydrid zu acetylieren. Dabei entsteht ein Gemisch aus ca. 28% 2-Acetat, 17% 5-Acetat und 57% 2,5-Diacetat, welches chromatographisch getrennt wird. Eine technische Synthese von Isosorbid-5-nitrat läßt sich nach dieser Methode nicht wirtschaftlich durchführen, da die chromatographische Trennung zu teuer ist und für eine gemeinsame Weiterverarbeitung das Gemisch zuviel 5-Acetat enthält (2-Acetat : 5-Acetat = ca. 1,6).

Aufgabe dieser Erfindung war es daher, ein Verfahren bereitzustellen, mit dem man Isosorbid-5-nitrat einfach und wirtschaftlich in großtechnischem Maßstab herstellen kann. Dabei war es insbesondere ein Anliegen, das Auftreten von nicht mehr aufarbeitbaren, komplexen Lösungsmittelgemischen zu vermeiden, da diese zu Beseitigungs- und damit Umweltproblemen führen.

Diese Aufgabe wird erfindungsgemäß dadurch gelöst, daß man ein Verfahren, bei dem man

a) 1,4:3,6-Dianhydro-D-glucit mit 0,5 bis 1,5 Moläquivalenten Essigsäureanhydrid in Pyridin acetyliert und aus dem Reaktionsgemisch 1,4:3,6-Dianhydro-D-glucit-2-acetat isoliert,

b) dieses mit Salpetersäure zu 1,4:3,6-Dianhydro-D-glucit-2-acetat-5-nitrat nitriert und

c) das gemäß Verfahrensstufe b) erhaltene Produkt mit einer anorganischen Base zu 1,4:3,6-Dianhydro-D-glucit-5-nitrat hydrolysiert,

wie es beim Stand der Technik unter Punkt d) dargestellt ist, dahingehend abwandelt, daß bei der Stufe

a) in einem Gemisch aus einem inerten, mit Wasser nicht mischbaren Lösungsmittel und einem basischen Lösungsmittel als Säureakzeptor und Katalysator bei Temperaturen zwischen 0 und 80°C acetyliert wird und vor der Umsetzung (b) aus dem vorstehenden Gemisch, das nichtumgesetzte 1,4:3,6-Dianhydro-D-glucit und das basische Lösungsmittel durch Waschen mit Wasser beziehungsweise wäßriger Säure abtrennt und das inerte Lösungsmittel sowie mitentstandenes 1,4:3,6-Dianhydro-D-glucit-2,5-diacetat durch Destillieren und Filtrieren weitgehend entfernt werden,

und bei der Stufe

b) das gemäß Verfahrensstufe a) erhaltene Acetatgemisch ohne Isolierung des 1,4:3,6-Dianhydro-D-glucit-2-acetats nitriert und das 1,4:3,6-Dianhydro-D-glucit-2-acetat-5-nitrat isoliert wird, indem man es durch Verdünnen der Nitriersäure mit Wasser ausfällt und anschließend abfiltriert.

Dabei hat die Verwendung des Lösungsmittel/Base-Gemisches der Stufe a) überraschenderweise zur Folge, daß die Menge an 1,4:3,6-Dianhydro-D-glucit-2-acetat erheblich größer ist als die des entsprechenden 5-Acetats, so daß das erhaltene Acetat-Gemisch ohne weitere Auftrennung in die Nitrierungsstufe eingesetzt werden kann.

Insofern ist auch bei der in der zweiten Stufe erfolgenden Nitrierung der Anteil an dem 1,4:3,6-Dianhydro-D-glucit-2-acetat-5-nitrat größer als der des entsprechenden 5-Acetat-2-nitrats. Überraschenderweise gelingt es darüber hinaus durch einen einfachen Fällungsprozeß mit Wasser das gewünschte 2-Acetat-5-nitrat fast rein abzutrennen.

Die in Stufe a) einzusetzenden Lösungsmittel müssen mit Wasser nicht mischbar sein, um bei der späteren Aufarbeitung leicht abgetrennt und wiedergewonnen zu werden und selbstverständlich inert gegenüber den anderen Reaktionskomponenten sein, damit sie nicht in den Reaktionsablauf eingreifen. Solche Lösungsmittel sind zum Beispiel Methylenchlorid, Chloroform, Benzol, Toluol oder Cyclohexan. Besonders bevorzugt ist Methylenchlorid.

Zur Bindung der in der Stufe a) entstehenden Essigsäure und als basischer Katalysator wird Pyridin, Triethylamin, oder Picolin, insbesondere Pyridin zugefügt.

In einer besonders bevorzugten Ausführungsform wird in der Stufe a) 1,4:3,6-Dianhydro-D-glucit mit 0,5 bis 1,5 Moläquivalenten Essigsäureanhydrid in einem Gemisch aus Methylenchlorid und Pyridin umgesetzt und vor der Umsetzung b) das Pyridin durch Waschen mit Schwefelsäure, das nichtumgesetzte 1,4:3,6-Dianhydro-D-glucit durch Waschen mit Wasser, das Methylenchlorid durch Abdestillieren und das 1,4:3,6-Dianhydro-D-glucit-2,5-diacetat nach Ausfällen durch Filtration entfernt, wobei man das Ausfällen des 1,4:3,6-Dianhydro-D-glucit-2,5-diacetats durch Animpfen, das heißt durch Zugabe weniger Kristalle reinen Diacetats zum eingeengten Produktgemisch überraschend einfach bewerkstelligt.

Bei dieser Arbeitsweise werden durch einfache, großtechnisch durchführbare Operationen

- beide Lösungsmittel, nichtumgesetztes Ausgangsprodukt sowie das entstandene Nebenprodukt zurückerhalten,
- in die Nitrierstufe b) die beiden interessierenden Monoacetate, vom Diacetat abgetrennt, eingebracht und somit
- das Auftreten von nicht mehr oder nur noch schwer auftrennbaren Lösungsmittelgemischen vermieden.

Das zurückgewonnene 1,4:3,6-Dianhydro-D-glucit-2,5-diacetat kann in einer weiteren bevorzugten Ausführungsform durch Filtration über einen Ionenaustauscher in der H+-Form in 1,4:3,6-Dianhydro-D-glucit zurückverwandelt und erneut in das Verfahren eingebracht werden, was einen erheblichen wirtschaftlichen Vorteil bedeutet.

Als besonders überraschend ist es anzusehen, daß es in der Stufe b) nach der Nitrierung möglich ist, durch einfaches Verdünnen der Nitriersäure mit Wasser das gewünschte 1,4:3,6-Dianhydro-D-glucit-2-acetat-5-nitrat in hoher Reinheit auszufällen und durch Abfiltrieren vom Nebenprodukt 1,4:3,6-Dianhydro-D-glucit-5-acetat-2-nitrat zu trennen.

Die auf diese Weise separierten stellungsisomeren 1,4:3,6-Dianhydro-D-glucit-Derivate können durch Hydrolyse nun nahezu quantitativ in das gewünschte 1,4:3,6-Dianhydro-D-glucit-5-nitrat bzw. das als Verfahrensnebenprodukt anfallende 1,4:3,6-Dianhydro-D-glucit-2-nitrat umgewandelt werden.

Das Verfahren wird anhand des folgenden Beispiels näher erläutert; es gibt eine der möglichen Varianten des Erfindungsgegenstandes wieder, soll ihn jedoch in keiner Weise einschränken:

Beispiel

Stufe a

Acetylierung von 1,4:3,6-Dianhydro-D-glucit

4,68 kg Isosorbid, 3,45 l Pyridin und 80 l Methylenchlorid werden vorgelegt und bei Raumtemperatur so lange gerührt, bis Lösung eingetreten ist. Danach werden 3,80 l Essigsäureanhydrid hinzugegeben, wobei keine merkliche Wärmetönung eintritt. Das Reaktionsgemisch wird 48 Stunden bei Raumtemperatur stehengelassen. Eine anschließende gaschromatographische Untersuchung ergibt folgende Zusammensetzung: 50,2% 1,4:3,6-Dianhydro-D-glucit-2-acetat, 6,3% 1,4:3,6-Dianhydro-D-glucit-5-acetat, 30,3% 1,4:3,6-Dianhydro-D-glucit-2,5-diacetat und 12,6% 1,4:3,6-Dianhydro-D-glucit. Die Methylenchloridphase wird anschließend mit verdünnter Schwefelsäure extrahiert (1,16 l conc. Schwefelsäure in 3,30 kg Eis). Nach dem Abtrennen der Schwefelsäurephase wird die Methylenchloridphase noch zweimal mit je 1,10 l Eiswasser gewaschen. Das Waschwasser enthält 1,4:3,6-Dianhydro-D-glucit und wird — wie weiter unten beschrieben — zusammen mit dem Diacetat aufgearbeitet. Die Methylenchloridphase wird nun bei 50°C und 400 Torr. zum Sirup eingeengt, wobei gegen Ende der Destillation für 2 Stunden die Badtemperatur auf 70°C erhöht und volles Wasserstrahlvakuum angelegt wird, um die Reste von Essigsäure und Pyridin abzutrennen. Man erhält 5,5 kg Produktgemisch, das nach gaschromatographischer Analyse 54,2% 1,4:3,6-Dianhydro-D-glucit-2-acetat, 6,2% 1,4:3,6-Dianhydro-D-glucit-5-acetat, 37,4% 1,4:3,6-Dianhydro-D-glucit-2,5-diacetat und 1,7% 1,4:3,6-Dianhydro-D-glucit enthält. Der 1,4:3,6-Dianhydro-D-glucit-Anteil kann durch weiteres Waschen mit Wasser vor Abziehen des Methylenchlorids weiter reduziert werden. Das erhaltene Produktgemisch wird mit 3,5 l Eiswasser versetzt. Nach vollständiger Mischung wird mit Diacetat angeimpft.

Nach Stehen bei 0 bis 5°C über Nacht kristallisiert ein Großteil des Diacetats (1 kg) aus, wird abgesaugt und mit 0,5 l Eiswasser gewaschen, wobei das Waschwasser mit dem Original-Filtrat vereinigt wird. Die vereinigten Filtrate werden im Vakuum bis zur Gewichtskonstanz eingeengt, wobei man 4,1 kg wasserfreies Acetatgemisch von 67,2% 1,4:3,6-Dianhydro-D-glucit-2-acetat, 7,7% 1,4:3,6-Dianhydro-D-glucit-5-acetat, 21,6% 1,4:3,6-Dianhydro-D-glucit-2,5-diacetat und 1,3% 1,4:3,6-Dianhydro-D-glucit (gaschromatographisch bestimmt) erhält.

Das abgetrennte Diacetat wird mit den 1,4:3,6-Dianhydro-D-glucit-haltigen Waschwässern vereinigt, auf 5 l mit Wasser aufgefüllt und bei 90°C in Lösung gebracht. Diese Lösung passiert bei 90°C eine Kationenaustauschersäule in der H+-Form mit einer Verweilzeit von 2 Stunden (Austauscher: z. B. SP 112 von Bayer). Das Eluat wird zu einem hellgelben Sirup (1 kg) eingeengt, der aus reinem 1,4:3,6-Dianhydro-D-glucit besteht und beim Stehen durchkristallisiert.

Der in obigem Verfahren erhaltene schwefelsaure Pyridinauszug wird mit 7,5 l verdünnter Natronlauge (2,5 l conc. NaOH in 5 l Wasser) versetzt, bis ein pH-Wert von 7 bis 8 erreicht ist. Das Pyridin scheidet sich als Oberphase ab, wird abgetrennt, über NaOH-Schuppen (0,7 kg) getrocknet und anschließend destilliert.

### Stufe b

#### 1,4:3,6-Dianhydro-D-glucit-2-acetat-5-nitrat

Ein Gemisch aus 5,1 l Eisessig und 4,8 l Acetanhydrid wird in einem Kältebad (−5°C) auf +2°C abgekühlt. Man tropft bei einer Innentemperatur von maximal +6°C 1,08 l hochkonzentrierte Salpetersäure ein. Das Nitriergemisch wird 5 Minuten bei +4°C Innentemperatur nachgerührt und dann innerhalb von ca. 1/2−1 Stunde mit einer Lösung von 3,84 kg Acetatgemisch aus Stufe a in 7,6 l Eisessig bei einer Innentemperatur zwischen 0 und +4°C versetzt. Man spült mit 2,6 l Eisessig nach, rührt eine Stunde im Kältebad (−2°C) und verdünnt dann mit 145 l Wasser. Dabei kristallisiert sofort das 1,4:3,6-Dianhydro-D-glucit-2-acetat-5-nitrat aus. Nach Rühren über Nacht bei 0°C saugt man ab und erhält 3,1 kg des nahezu reinen Produktes. Dieses Produkt wird ohne weitere Reinigung direkt in die nächste Stufe eingesetzt.

### Stufe c

#### 1,4:3,6-Dianhydro-D-glucit-5-nitrat

3,1 kg 1,4:3,6-Dianhydro-D-glucit-2-acetat-5-nitrat (Stufe b) werden in 24 l Wasser von Raumtemperatur suspendiert. Unter Einhaltung eines pH-Wertes zwischen 10 und 12 werden 530 ml 40%ige Natronlauge zugegeben. Die Beendigung der Verseifung erkennt man daran, daß der pH-Wert nicht mehr fällt, weitgehend alles gelöst ist und im DC (Laufmittel $CH_3OH$/$CH_2Cl_2$ 4/96; Detektion mit schwefelsaurer Diphenylaminlösung) kein Ausgangsmaterial mehr nachweisbar ist. Die Reaktionslösung wird mit 10 ml conc. Schwefelsäure auf pH 6,8 bis 7 eingestellt und dann mit 8,7 kg NaCl versetzt. Dabei fällt 1,4:3,6-Dianhydro-D-glucid-5-nitrat als voluminöse Fällung aus. Es wird einmal mit 12 l und zweimal mit je 8 l $CH_2Cl_2$ extrahiert; die vereinigten Methylenchloridextrakte über Natriumsulfat getrocknet und eingeengt. Es resultiert ein glasiger Rückstand. Dieser glasige Rückstand wird mit 28 l Benzin (Siedepunkt 40−60°C) bei Raumtemperatur gerührt, wobei das Produkt kristallin wird und pulvrig ausfällt. Das Produkt wird abgesaugt und bei Raumtemperatur getrocknet.

Ausbeute: 1,815 kg
Fp: 92/94−96°
$\alpha_D^{20}$ = +174,9 c = 1% in $CH_3OH$
DC: $CH_3OH$/$CH_2Cl_2$ 4/96; Ligroin-Ether-Eisessig) einheitlich
HPLC: 100%ig

Es ist zu beachten, daß das kristallisierte, trockene 1,4:3,6-Dianhydro-D-glucit-5-nitrat in die Klasse der Sprengstoffe eingereiht werden muß und deshalb mit den entsprechenden Vorsichtsmaßnahmen zu handhaben ist. In der Praxis — und dies trifft für eine Tonnen-Produktion zu — ist es jedoch nicht notwendig, das Produkt in kristalliner Form, wie vorstehend beschrieben, zu isolieren.

Großtechnisch wird bevorzugt wie folgt verfahren: Der in Stufe c erwähnte glasige Rückstand wird in Aceton gelöst und die acetonische Lösung wird dann auf Lactose aufgesprüht. Nach dem Trocknen erhält man eine sogenannte »phlegmatisierte Masse«, die aus einem Teil 1,4:3,6-Dianhydro-D-glucit-5-nitrat und vier Teilen Lactose besteht. In dieser ungefährlichen Form gelangt das Produkt zur weiteren Verarbeitung, beispielsweise in Tablettenform. Selbstverständlich können auch andere zur Herstellung einer phlegmatisierten Masse geeignete Hilfsstoffe verwendet werden.

## Patentansprüche

1. Verfahren zur Herstellung von 1,4:3,6-Dianhydro-D-glucit-5-nitrat, bei dem man

a) 1,4:3,6-Dianhydro-D-glucit mit 0,5 bis 1,5 Moläquivalenten Essigsäureanhydrid in Pyridin acetyliert und aus dem Reaktionsgemisch 1,4:3,6-Dianhydro-D-glucit-2-acetat isoliert,
b) dieses mit Salpetersäure zu 1,4:3,6-Dianhydro-D-glucit-2-acetat-5-nitrat nitriert und
c) das gemäß Verfahrensstufe b) erhaltene Produkt mit einer anorganischen Base zu 1,4:3,6-Dianhydro-D-glucit-5-nitrat hydrolysiert,

dadurch gekennzeichnet, daß bei der Stufe

a) in einem Gemisch aus einem inerten, mit Wasser nicht mischbaren Lösungsmittel und einem basischen Lösungsmittel als Säureakzeptor und Katalysator bei Temperaturen zwischen 0 und 80°C acetyliert wird und vor der Umsetzung b) aus dem vorstehenden Gemisch, das nichtumgesetzte 1,4:3,6-Dianhydro-D-glucit und das basische Lösungsmittel durch Waschen mit Wasser beziehungsweise wäßriger Säure abtrennt und das inerte Lösungsmittel sowie mitentstandenes 1,4:3,6-Dianhydro-D-glucit-2,5-diacetat durch Destillieren und Filtrieren weitgehend entfernt werden,
und bei der Stufe
b) das gemäß Verfahrensstufe a) erhaltene Acetatgemisch ohne Isolierung des 1,4:3,6-Dianhydro-D-glucit-2-acetats nitriert und das 1,4:3,6-Dianhydro-D-glucit-2-acetat-5-nitrat isoliert wird, indem man es durch Verdünnen der Nitriersäure mit Wasser ausfällt und anschließend abfiltriert.

2. Verfahren nach Anspruch 1, dadurch gekennzeichnet, daß bei der Stufe

a) in einem Gemisch aus Methylenchlorid und Pyridin gearbeitet wird.

3. Verfahren nach einem der Ansprüche 1 und 2, dadurch gekennzeichnet, daß das abgetrennte 1,4:3,6-Dianhydro-D-glucit-2,5-diacetat durch Filtration über einen Ionenaustauscher in der H⁺-Form in 1,4:3,6-Dianhydro-D-glucit zurückverwandelt und erneut in das Verfahren eingebracht wird.

4. Verfahren nach einem der Ansprüche 1 bis 3, dadurch gekennzeichnet, daß man nach der Stufe c) das 1,4:3,6-Dianhydro-D-glucit-5-nitrat durch Extrahieren mit einem inerten Lösungsmittel und anschließendes Einengen weitgehend rein isoliert und anschließend gegebenenfalls durch Aufsprühen auf einen geeigneten Hilfsstoff eine phlegmatisierte Form herstellt.

## Claims

1. Process for the preparation of 1,4:3,6-dianhydro-D-glucitol-5-nitrate, in which one

a) acetylates 1,4:3,6-dianhydro-D-glucitol with 0.5 to 1.5 mole equivalents of acetic anhydride in pyridine and isolates 1,4:3,6-dianhydro-D-glucitol-2-acetate,
b) nitrates this with nitric acid to give 1,4:3,6-dianhydro-D-glucitol-2-acetate-5-nitrate and
c) hydrolyses the product obtained according to process step b) with an inorganic base to give 1,4:3,6-dianhydro-D-glucitol-5-nitrate,

characterised in that in the case of step

a) one acetylates in a mixture of an inert, waterimmiscible solvent and a basic solvent as acid acceptor and catalyst at temperatures between 0 and 80°C. and, before reaction b), separates from the mixture present the unreacted 1,4:3,6-dianhydro-D-glucitol and the basic solvent by washing with water or aqueous acid and substantremoves the inert solvent as well as simultaneously formed 1,4:3,6-dianhydro-D-glucitol-2,5-diacetate by distillation and filtration,
and in the case of step
b) nitrates the acetate mixture obtained according to step a) without isolation of the 1,4:3,6-dianhydro-D-glucitol-2-acetate and isolates the 1,4:3,6-dianhydro-D-glucitol-2-acetate-5-nitrate in that one precipitates it out by dilution of the nitrating acid with water and subsequently filters off.

2. Process according to claim 1, characterised in that, in the case of step a), one operates in a mixture of methylene chloride and pyridine.

3. Process according to one of claims 1 and 2, characterised in that the separated 1,4:3,6-dianhydro-D-glucitol-2,5-diacetate is converted back into 1,4:3,6-dianhydro-D-glucitol by filtration through an ion exchanger in the H+ form and again introduced into the process.

4. Process according to one of claims 1 to 3, characterised in that, after step c), one isolates the 1,4:3,6-dianhydro-D-glucitol-5-nitrate substantially pure by extraction with an inert solvent and subsequent evaporation and subsequently, if desired, produces a desensitised form by spraying on to an appropriate adjuvant.

## Revendications

1. Procédé de préparation du 5-nitrate de 1,4:3,6-dianhydro-D-glucitol, dans lequel

a) on soumet le 1,4:3,6-dianhydro-D-glucitol à une acétylation avec 0,5 à 1,5 équivalent-mole d'anhydride acétique dans de la pyridine et on isole à partir du mélange réactionnel le 2-acétate de 1,4:3,6-dianhydro-D-glucidol,
b) on soumet ce dernier à une nitration avec de l'acide nitrique pour le transformer en 2-acétate 5-nitrate de 1,4:3,6-dianhydro-D-glucitol, et
c) on hydrolyse le produit obtenu à l'issue du stade b) avec une base minérale pour obtenir le 5-nitrate de 1,4:3,6-dianhydro-D-glucitol,

caractérisé en ce que, dans le stade

a) on effectue l'acétylation dans un mélange comprenant un solvant inerte non miscible à l'eau et un solvant basique comme accepteur d'acide et catalyseur, à une température entre 0 et 80°C, on isole de ce mélange, avant d'effectuer la réaction du stade b) le 1,4:3,6-dianhydroglucitol non transformé et le solvant basique par des lavages avec de l'eau respectivement de l'acide aqueux, et on élimine par distillation et filtration pratiquement tout le solvant inerte ainsi que le 2,5-diacétate de 1,4:3,6-dianhydro-D-glucitol également formé,

et dans le stade

b) on soumet le mélange d'acétates obtenu selon le stade a) sans isoler le 2-acétate de 1,4:3,6-dianhydro-D-glucitol, à une nitration et on isole le 2-acétate 5-nitrate de 1,4:3,6-dianhydro-D-glucitol en le précipitant par dilution de l'acide de nitration avec de l'eau et en le filtrant ensuite.

2. Procédé selon la revendication 1, caractérisé en ce que dans le stade

a) on travaille dans un mélange de chlorure de méthylène et de pyridine.

3. Procédé selon l'une des revendications 1 et 2, caractérisé en ce que le 2,5-diacétate de 1,4:3,6-dianhydro-D-glucitol séparé est retrans-formé en 1,4:3,6-dianhydro-D-glucitol par filtration sur un échangeur d'ions en forme H+ et est recyclé dans le procédé.

4. Procédé selon l'une des revendications 1 à 3, caractérisé en ce qu'après le stade c) on isole le 5-nitrate de 1,4:3,6-dianhydro-D-glucitol à l'état pratiquement pur, par extraction avec un solvant inerte et concentration consécutive et, on prépare éventuellement ensuite, par étinceler sur une substance auxiliaire, une forme phleg-matisée.